(19) Europäisches Patentamt
European Patent Office
Office européen des brevets

(11) **EP 3 977 926 A1**

(12) **EUROPEAN PATENT APPLICATION**
published in accordance with Art. 153(4) EPC

(43) Date of publication:
**06.04.2022 Bulletin 2022/14**

(51) International Patent Classification (IPC):
**A61B 5/117** *(2016.01)*

(21) Application number: **19930760.4**

(52) Cooperative Patent Classification (CPC):
**A61B 5/117**

(22) Date of filing: **29.05.2019**

(86) International application number:
**PCT/JP2019/021422**

(87) International publication number:
**WO 2020/240751 (03.12.2020 Gazette 2020/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **NEC Corporation**
**108-8001 Tokyo (JP)**

(72) Inventors:
• **HUANG, Chenhui**
  **Tokyo 108-8001 (JP)**
• **FUKUSHI, Kenichiro**
  **Tokyo 108-8001 (JP)**
• **NAKAHARA, Kentaro**
  **Tokyo 108-8001 (JP)**

(74) Representative: **Betten & Resch**
**Patent- und Rechtsanwälte PartGmbB**
**Maximiliansplatz 14**
**80333 München (DE)**

(54) **INFORMATION PROCESSING DEVICE, INDIVIDUAL IDENTIFICATION DEVICE, INDIVIDUAL IDENTIFICATION SYSTEM, INFORMATION PROCESSING METHOD AND STORAGE MEDIUM**

(57) Provided is an information processing device including an acquisition unit configured to acquire motion information of a foot of a user measured by a motion measurement device provided on the foot and a feature amount extracting unit configured to extract a feature amount used for identifying the user from time series data of at least one walking cycle included in the motion information.

FIG. 4

**Description**

[Technical Field]

The present invention relates to an information processing device, a personal identification device, a personal identification system, an information processing method, and a storage medium.

[Background Art]

Patent Literature 1 discloses an admission management system for performing personal authentication using acceleration detected by an acceleration sensor mounted on a portable terminal such as a smartphone. Patent Literature 2 discloses that a habit of walking detected by a load sensor, an acceleration sensor, a Hall element, or the like mounted on an insole for a shoe can be used for personal identification.

[Citation List]

[Patent Literature]

PTL 1: Japanese Patent Application Laid-open No. 2015-153143
PTL 2: Japanese Patent Application Laid-open No. 2016-73366

[Non Patent Literature]

NPL 1: Sebastian O. H. Madgwick, Andrew J. L. Harrison, and Ravi Vaidyanathan, "Estimation of IMU and MARG orientation using a gradient descent algorithm, " 2011 IEEE International Conference on Rehabilitation Robotics, pp. 179-185, 2011.

[Summary of Invention]

[Technical Problem]

In personal identification using motion information during walking as disclosed in Patent Literature 1 or Patent Literature 2, a more preferable method of extracting a feature amount is required.
The present invention intends to provide an information processing device, a personal identification device, a personal identification system, an information processing method, and a storage medium which can suitably extract a feature amount used for personal identification.

[Solution to Problem]

According to one example aspect of the invention, provided is an information processing device including an acquisition unit configured to acquire motion information of a foot of a user measured by a motion measurement device provided on the foot and a feature amount extracting unit configured to extract a feature amount used for identifying the user from time series data of at least one walking cycle included in the motion information.
According to another example aspect of the invention, provided is an information processing method including acquiring motion information of a foot of a user measured by a motion measurement device provided on the foot and extracting a feature amount used for identifying the user from time series data of at least one walking cycle included in the motion information.
According to another example aspect of the invention, provided is a storage medium storing a program that causes a computer to perform acquiring motion information of a foot of a user measured by a motion measurement device provided on the foot and extracting a feature amount used for identifying the user from time series data of at least one walking cycle included in the motion information.

[Advantageous Effects of Invention]

According to the present invention, a personal identification device, a personal identification system, an information processing method, and a storage medium which can suitably extract a feature amount used for personal identification can be provided.

[Brief Description of Drawings]

[Fig. 1]

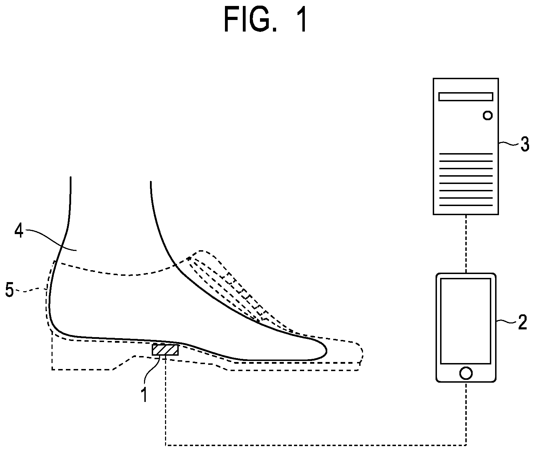

Fig. 1 is a schematic diagram illustrating a general configuration of a personal identification system according to a first example embodiment.
[Fig. 2]

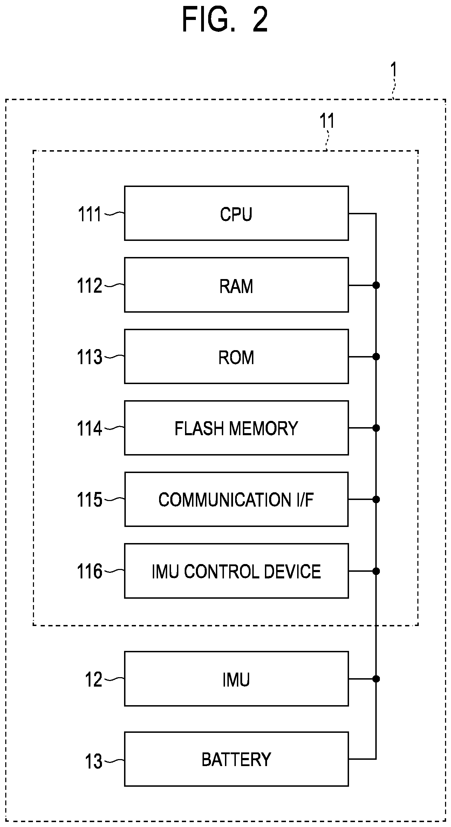

Fig. 2 is a block diagram illustrating a hardware configuration of a gait measurement device according to the first example embodiment.
[Fig. 3]

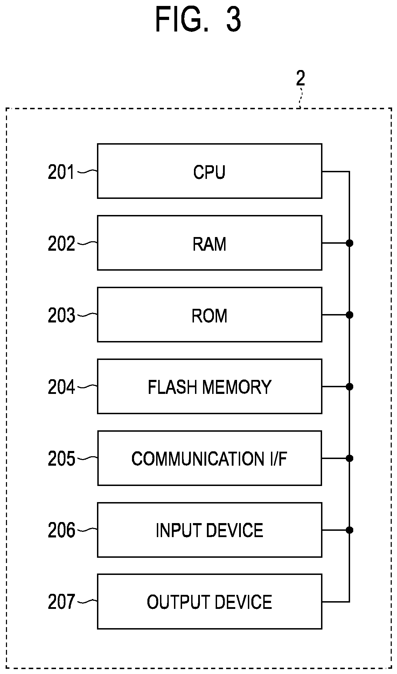

Fig. 3 is a block diagram illustrating a hardware configuration of an information communication terminal according to the first example embodiment.
[Fig. 4]

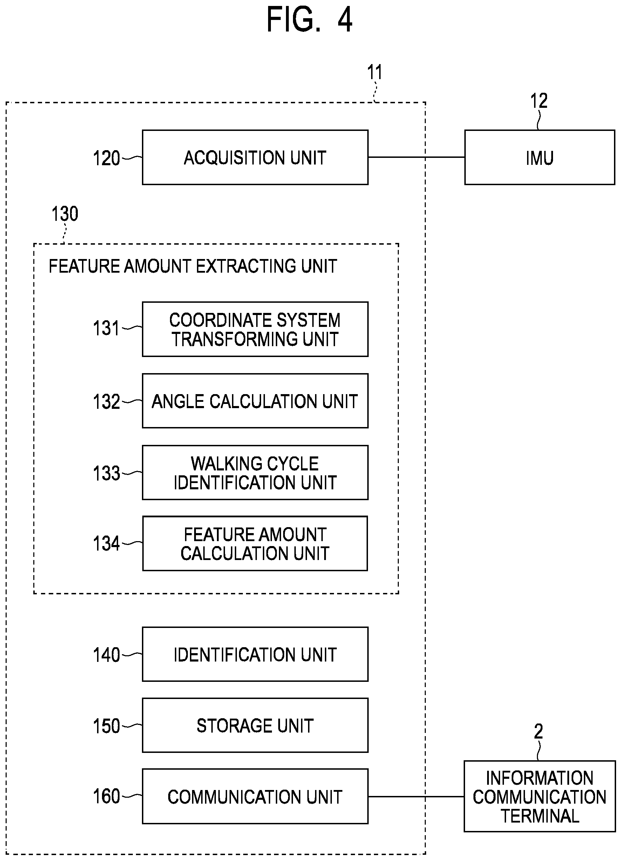

Fig. 4 is a functional block diagram of an information processing device according to the first example embodiment.
[Fig. 5]

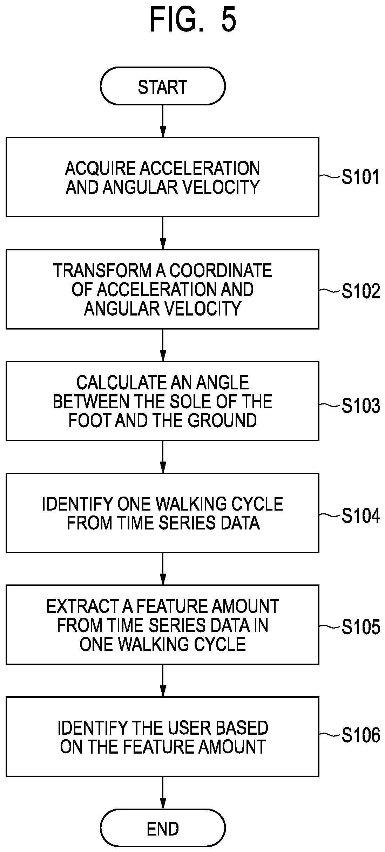

Fig. 5 is a flowchart illustrating an example of personal identification processing performed by the gait measurement device according to the first example embodiment.
[Fig. 6]

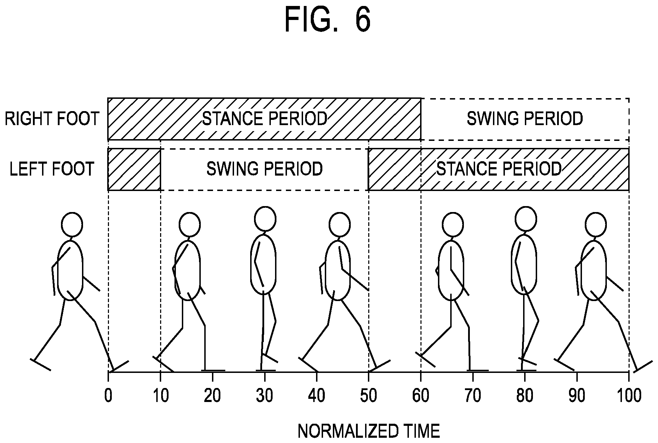

Fig. 6 is a conceptual diagram illustrating a walking cycle.
[Fig. 7]

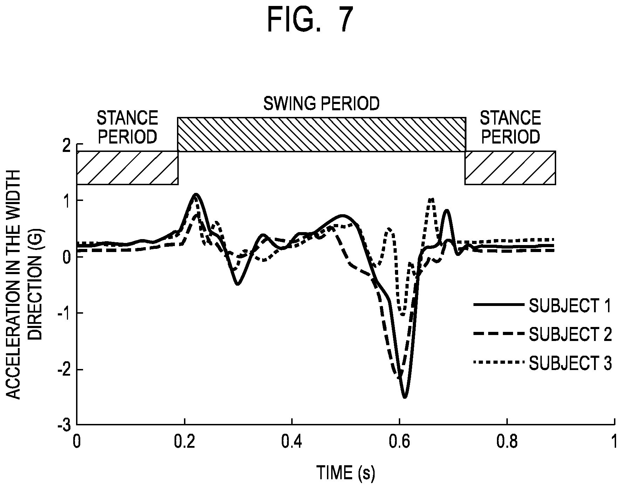

Fig. 7 is a graph illustrating an example of time series data of acceleration in the width direction of the foot.
[Fig. 8]

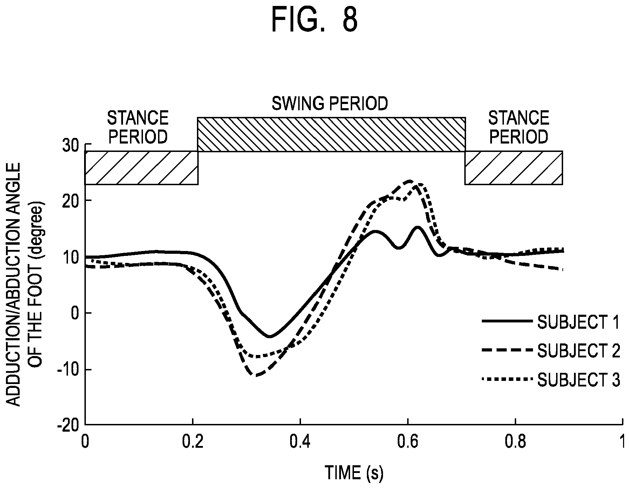

Fig. 8 is a graph illustrating an example of time series data of an adduction/abduction angle of the foot.
[Fig. 9]

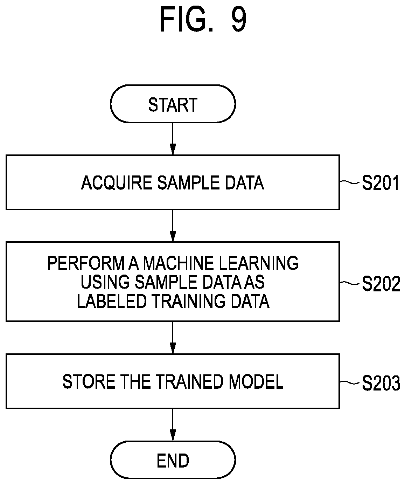

Fig. 9 is a flowchart illustrating an example of training processing performed by the server according to the first example embodiment.
[Fig. 10]

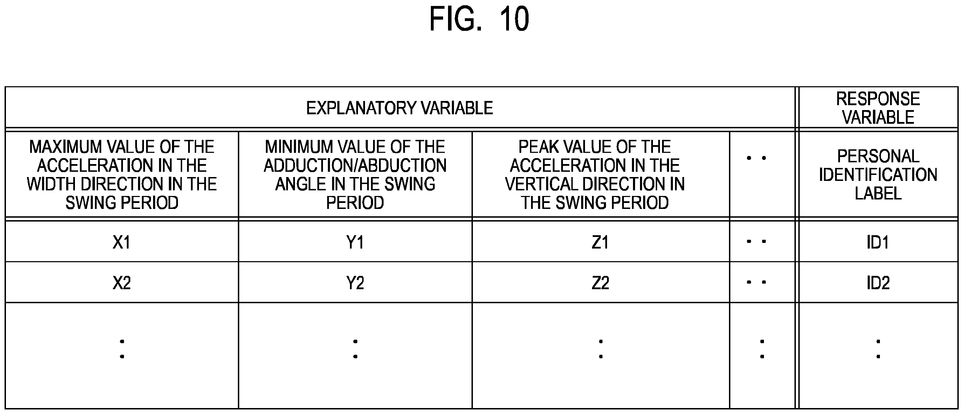

Fig. 10 is a table schematically illustrating a correspondence relation between a feature amount vector and a personal identification label acquired by the training processing.
[Fig. 11]

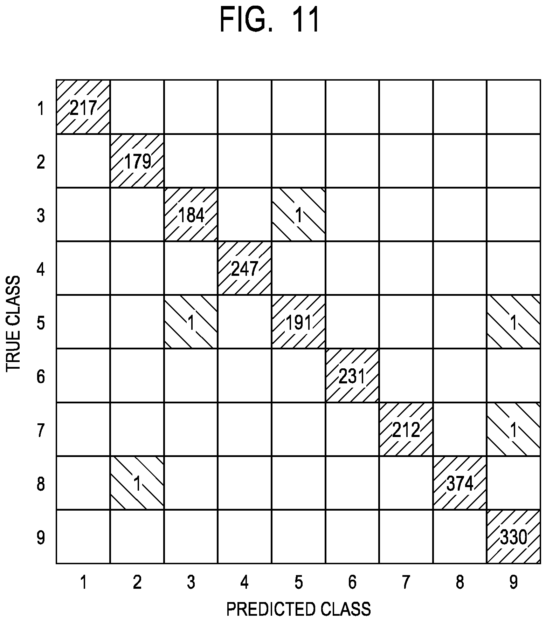

Fig. 11 is a table illustrating a result of cross-validation.
[Fig. 12]

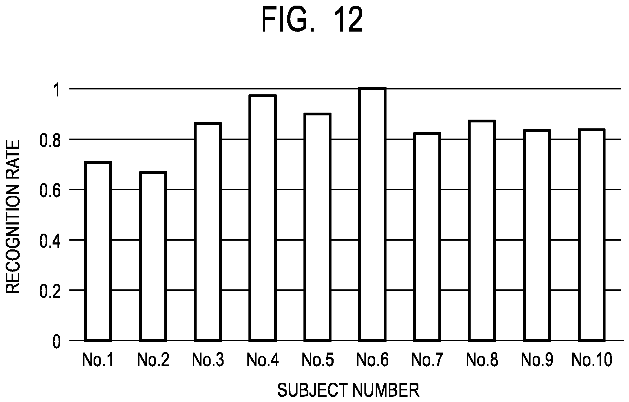

Fig. 12 is a graph illustrating a result of validation of a recognition rate.
[Fig. 13]

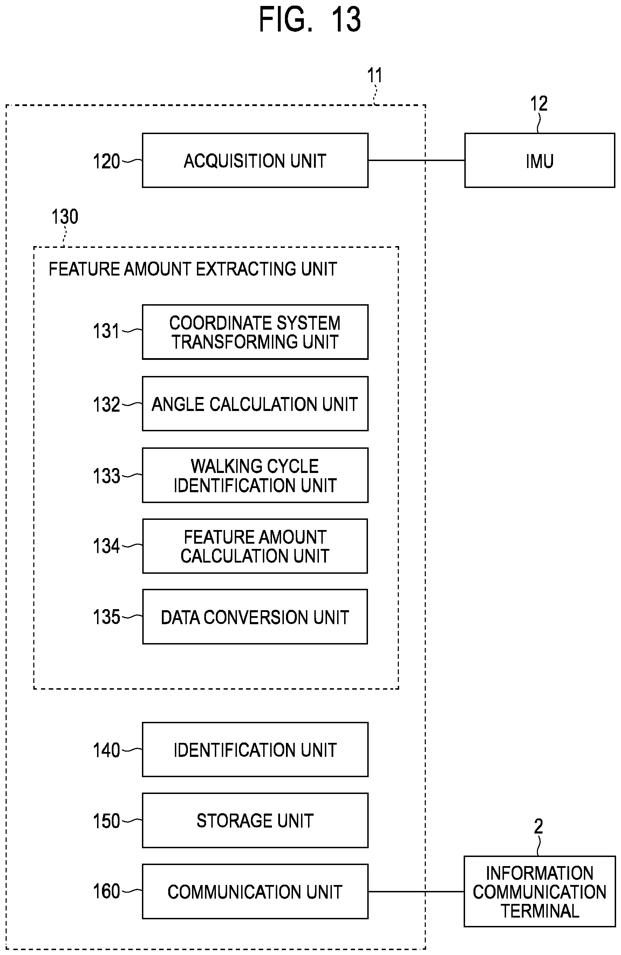

Fig. 13 is a functional block diagram of an information processing device according to a second example embodiment.
[Fig. 14]

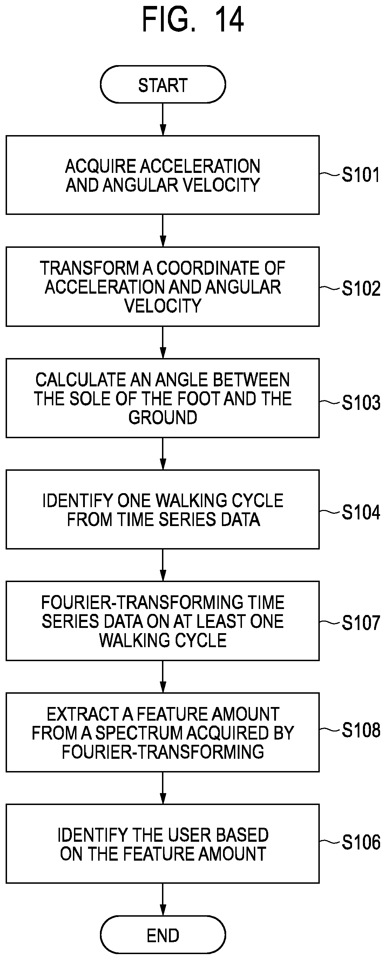

Fig. 14 is a flowchart illustrating an example of personal identification processing performed by a gait measurement device according to the second example embodiment.
[Fig. 15]

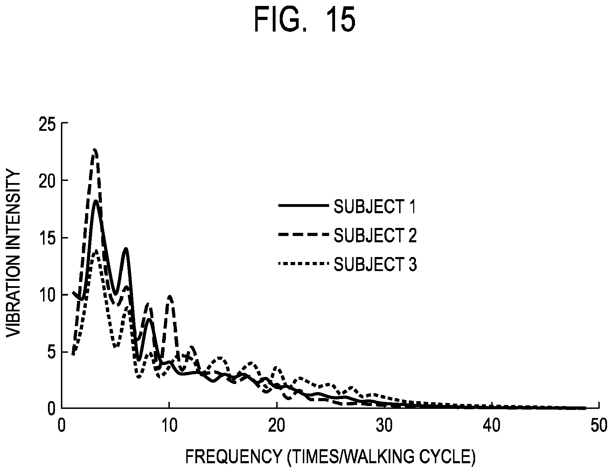

Fig. 15 is a graph illustrating an example of a frequency spectrum of acceleration in the width direction of the foot.
[Fig. 16]

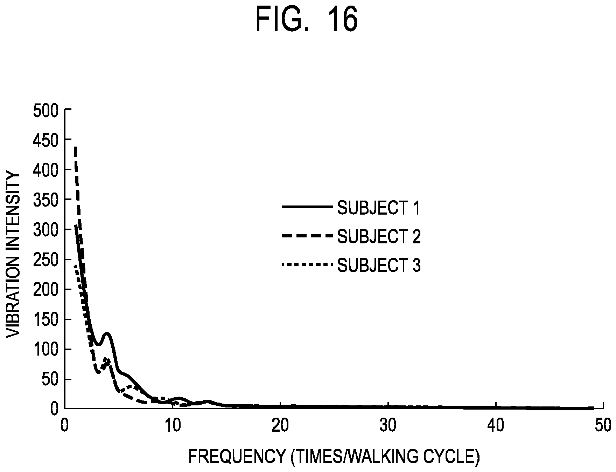

Fig. 16 is a graph illustrating an example of a frequency spectrum of an adduction/abduction angle of the foot.
[Fig. 17]

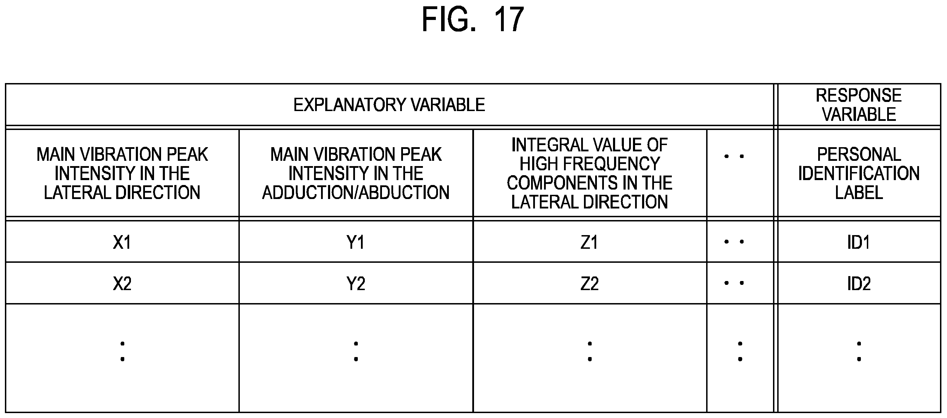

Fig. 17 is a table schematically illustrating a correspondence relation between a feature amount vector and a personal identification label acquired by the training processing.
[Fig. 18]

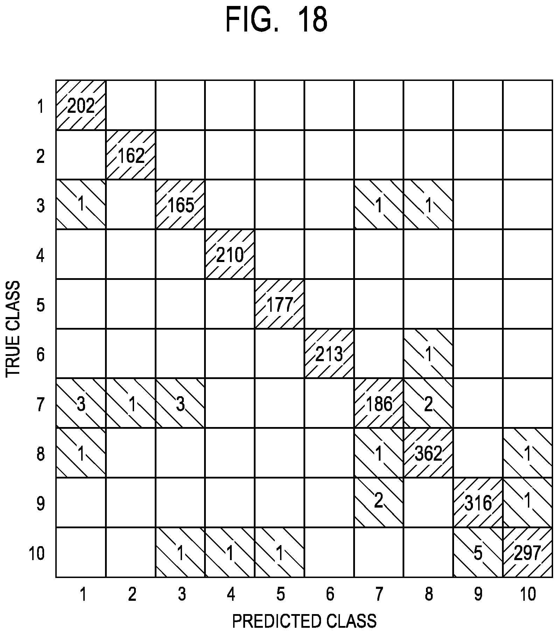

Fig. 18 is a table illustrating a result of cross-validation.
[Fig. 19]

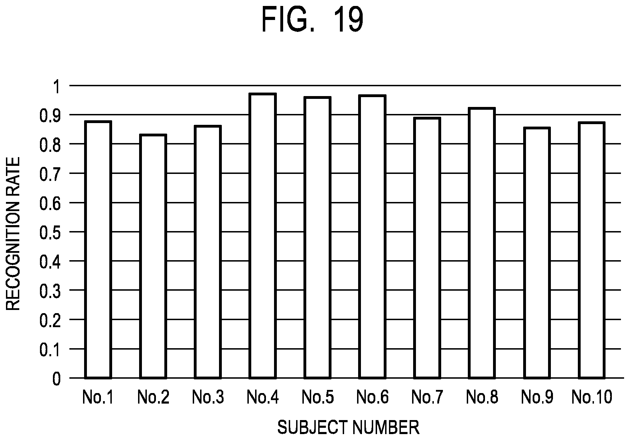

Fig. 19 is a graph illustrating a result of validation of a recognition rate.
[Fig. 20]

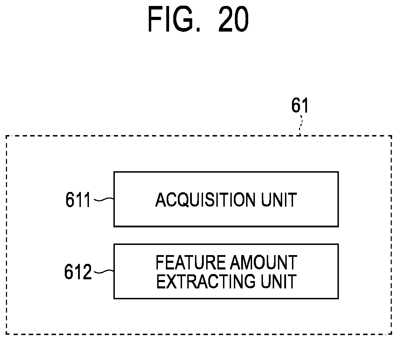

Fig. 20 is a functional block diagram of an information processing device according to a third example embodiment.

[Description of Embodiments]

Exemplary embodiments of the present invention are described below with reference to the drawings. Throughout the drawings, the same components or corresponding components are labeled with same references, and the description thereof may be omitted or simplified.

[First Example Embodiment]

A personal identification system according to the present example embodiment is described. The personal identification system of the present example embodiment is a system for performing personal identification by measuring and analyzing features included in a walking pattern of a user (gait).

Fig. 1 is a schematic diagram illustrating a general configuration of a personal identification system according to the present example embodiment. The personal identification system includes a gait measurement device 1, an information communication terminal 2, and a server 3 which may be connected to each other by wireless communication.

The gait measurement device 1 is provided to be close to the sole of a shoe 5 worn by a user 4, for example. The gait measurement device 1 is an electronic apparatus having a sensing function for measuring a motion of the foot of the user 4, an information processing function for analyzing the measured motion information, a communication function with the information communication terminal 2, and the like. It is desirable that the gait measurement device 1 be provided at a position corresponding to the arch of the foot such as just below the arch of the foot. In this case, the gait measurement device 1 can measure acceleration and angular velocity of the center of the foot of the user 4. Since the center of the foot is a position showing the feature of the motion of the foot well, it is suitable for feature amount extraction.

Note that, the gait measurement device 1 may be provided in the insole of the shoe 5, may be provided in the outsole of the shoe 5, or may be embedded in the shoe 5. The gait measurement device 1 may be detachably attached to the shoe 5 or may be non-detachably fixed to the shoe 5. The gait measurement device 1 may be provided at a portion other than the shoe 5 as long as the gait measurement device 1 can measure the motion of the foot. For example, the gait measurement device 1 may be provided in a sock which the user 4 is wearing, provided in a decoration, directly attached to the foot of the user 4, or embedded in the foot of the user 4. Although Fig. 1 illustrates an example in which one gait measurement device 1 is provided on one foot of the user 4, one gait measurement device 1 may be provided on each of both feet of the user 4. In this case, the motion information of both feet can be acquired in parallel, and more information can be acquired.

In this specification, the "foot" means a body part below an ankle of the user 4. In addition, in this specification, the "user" means a person who is an object of personal identification using the gait measurement device 1. Whether or not the user corresponds to the "user" is unrelated to whether or not the user is a user of a device other than the gait measurement device 1 constituting the personal identification system, whether or not the user receives a service provided by the personal identification system, or the like.

The information communication terminal 2 is a terminal device carried by the user 4, such as a cellular phone, a smartphone, or a smart watch. Application software for analyzing information acquired from the gait measurement device 1 is installed in advance in the information communication terminal 2, and processing based on the application software is performed. The information communication terminal 2 acquires data acquired by the gait measurement device 1 and performs information processing using the data. The result of the information processing may be notified to the user 4 or may be transmitted to the server 3. The information communication terminal 2 may have a function of providing software such as a control program of the gait measurement device 1 or a data analysis program to the gait measurement device 1.

The server 3 provides and updates analysis application software to the information communication terminal 2. The server 3 may store data acquired from the information communication terminal 2 and perform information processing using the data.

Note that, the general configuration is an example, and for example, the gait measurement device 1 may be directly connected to the server 3. Further, the gait measurement device 1 and the information communication terminal 2 may be configured as an integrated device, and another device such as an edge server or a relay device may be further included in the personal identification system.

Fig. 2 is a block diagram illustrating a hardware configuration example of the gait measurement device 1. The gait measurement device 1 includes an information processing device 11, an inertial measurement unit (IMU) 12, and a battery 13.

The information processing device 11 is, for example, a microcomputer or a microcontroller that performs a control of the entire gait measurement device 1 and data processing. The information processing device 11 includes a central processing unit (CPU) 111, a random access memory (RAM) 112, a read only memory (ROM) 113, a flash memory 114, a communication interface (I/F) 115, and an IMU control device 116. Each unit in the information processing device 11, the IMU 12, and the battery 13 is connected each other via a bus, wiring, a driving device, or the like.

The CPU 111 is a processor that performs predetermined calculation in accordance with a program stored in the ROM 113, the flash memory 114, or the like, and also has a function of controlling each unit of the information processing device 11. The RAM 112 is composed of a volatile storage medium and provides a temporary memory area required for the operation of the CPU 111. The ROM 113 is composed of a non-volatile storage medium and stores necessary information such as a program used for the operation of the information processing device 11. The flash memory 114 is a storage device composed of a non-volatile storage medium and temporarily storing data, storing an operation program of the information processing device 11, or the like.

The communication I/F 115 is a communication interface based on standards such as Bluetooth (registered trademark) or Wi-Fi (registered trademark), and is a module for performing communication with the information communication terminal 2.

The IMU 12 is a motion measurement device including an angular velocity sensor that measures angular velocity in three axial directions and an acceleration sensor that measures acceleration in three directions. The angular velocity sensor may be any sensor as long as it can acquire the angular velocity as time series data, and any type of sensor such as a vibration type sensor or a capacitance type sensor may be used. The acceleration sensor may be any type of sensor as long as it can acquire acceleration as time series data, and any type of sensor such as a piezoelectric type sensor, a piezoresistance type sensor, or a capacitance type sensor may be used. In the present example embodiment, the interval between the data points of the acquired time series data may be constant or may not be constant.

The IMU control device 116 is a control device that controls the IMU 12 to measure angular velocity and acceleration and acquires angular velocity and acceleration acquired by the IMU 12. The acquired angular velocity and acceleration are stored in the flash memory 114 as digital data. Note that analog-to-digital (AD) conversion for converting an analog signal measured by the IMU 12 into digital data may be performed in the IMU 12 or may be performed by the IMU control device 116.

The battery 13 is, for example, a secondary battery, and supplies power necessary for the operations of the information processing device 11 and the IMU 12. Since the battery 13 is built in the gait measurement device 1, the gait measurement device 1 can operate wirelessly without connecting to an external power source by wire.

Note that the hardware configuration illustrated in Fig. 2 is an example, and other devices may be added or some devices may not be provided. Further, some devices may be replaced by other devices having similar functions. For example, the information processing device 11 may further include an input device such as a button so that an operation by the user 4 can be accepted, and may further include an output device such as a display, a display lamp, and a speaker for providing information to the user 4. Thus, the hardware configuration illustrated in Fig. 2 can be changed appropriately.

Fig. 3 is a block diagram illustrating a hardware configuration example of the information communication terminal 2. The information communication terminal 2 includes a CPU 201, a RAM 202, a ROM 203, and a flash memory 204. The information communication terminal 2 also includes a communication I/F 205, an input device 206, and an output device 207. Each unit of the information communication terminal 2 is connected to each other via a bus, wiring, a driving device, or the like.

In Fig. 3, each unit constituting the information communication terminal 2 is illustrated as an integrated device, but a part of these functions may be provided by an external device. For example, the input device 206 and the output device 207 may be external devices different from those constituting the functions of the computer including the CPU 201 or the like.

The CPU 201 is a processor that performs predetermined calculation in accordance with a program stored in the ROM 203, the flash memory 204, or the like, and also has a function of controlling each unit of the information communication terminal 2. The RAM 202 is composed of a volatile storage medium and provides a temporary memory area required for the operation of the CPU 201. The ROM 203 is composed of a non-volatile storage medium and stores necessary information such as a program used for the operation of the information communication terminal 2. The flash memory 204 is a storage device composed of a non-volatile storage medium for storing data transmitted and received to and from the gait measurement device 1 and for storing a program for operating the information communication terminal 2.

The communication I/F 205 is a communication interface based on standards such as Bluetooth (registered trademark), Wi-Fi (registered trademark), or 4G and is a module for performing communication with other devices.

The input device 206 is a user interface used by the user 4 to operate the information communication terminal 2. Examples of the input device 206 include a mouse, a trackball, a touch panel, a pen tablet, a button, or the like.

The output device 207 is, for example, a display device. The display device is a liquid crystal display, an organic light emitting diode (OLED) display, or the like, and is used for displaying information, displaying a graphical user interface (GUI) for operation input, or the like. The input device 206 and the output device 207 may be integrally formed as a touch panel.

Note that the hardware configuration illustrated in Fig. 3 is an example, and other devices may be added or some devices may not be provided. Further, some devices may be replaced by other devices having similar functions. Further, some functions of the present example embodiment may be provided by other devices via a network, or some functions of the present example embodiment may be realized by being distributed among a plurality of devices. For example, the flash memory 204 may be replaced by a hard disk drive (HDD) or a cloud storage. Thus, the hardware configuration illustrated in Fig. 3 can be changed appropriately.

The server 3 is a computer having substantially the same hardware configuration as that illustrated in Fig. 3. Since the hardware configuration of the server 3 is substantially the same as that of the information communication terminal 2 except that the server 3 may not be portable, a detailed description thereof is omitted.

Fig. 4 is a functional block diagram of the information processing device 11 according to the present example embodiment. The information processing device 11 includes an acquisition unit 120, a feature amount extracting unit 130, an identification unit 140, a storage unit 150, and a communication unit 160. The feature amount extracting unit 130 includes a coordinate system transforming unit 131, an angle calculation unit 132, a walking cycle identification unit 133, and a feature amount calculation unit 134.

The CPU 111 loads a program stored in the ROM 113, the flash memory 114, or the like into the RAM 112 and executes the program. Thus, the CPU 111 realizes the functions of the feature amount extracting unit 130 and the identification unit 140. Further, the CPU 111 realizes the function of the acquisition unit 120 by controlling the IMU control device 116 based on the program. The CPU 111 realizes the function of the storage unit 150 by controlling the flash memory 114 based on the program. Further, the CPU 111 realizes the function of the communication unit 160 by controlling the communication I/F 115 based on the program. Specific processing performed by these units is described later.

In the present example embodiment, each function of the functional blocks illustrated in Fig. 4 is provided in the gait measurement device 1, but some functions of the functional blocks illustrated in Fig. 4 may be provided in the information communication terminal 2 or the server 3. That is, the above-described functions may be realized by any of the gait measurement device 1, the information communication terminal 2, and the server 3, or may be realized by cooperation of the gait measurement device 1, the information communication terminal 2, and the server 3. In the present example embodiment, since the gait measurement device 1 has a function of performing personal identification processing, the gait measurement device 1 may be referred to as a personal identification device more generally.

Fig. 5 is a flowchart illustrating an example of personal identification processing performed by the gait measurement device 1 according to the present example embodiment. The process of Fig. 5 is performed when the gait measurement device 1 detects walking, for example, when the user 4 is walking. Alternatively, the process of Fig. 5 may be always performed unrelated to whether or not the user 4 is walking, or may be performed at predetermined time intervals.

In step S101, the acquisition unit 120 controls the angular velocity sensor and the acceleration sensor of the IMU 12 to acquire time series data of angular velocity in three axial direction and acceleration in three directions. Thus, the acquisition unit 120 can acquire time changes in angular velocity and acceleration caused by walking of the user 4. The acquired time series data of angular velocity and acceleration is converted into digital data and then stored in the storage unit 150. These angular velocity and acceleration are referred to more generally as motion information. Among the motion information, time series data of angular velocity and acceleration during walking is particularly referred to as walking data. The walking data can be used not only for the personal identification processing of the present example embodiment but also for the gait analysis of the user 4.

The three directions of acceleration acquired by the acquisition unit 120 may be, for example, the width direction (left/right direction), the longitudinal direction (front/back direction), and the vertical direction of the foot of the user 4 provided with the IMU 12. These directions are referred to as x-axis, y-axis, and z-axis, respectively. The three axial directions of the angular velocities acquired by the acquisition unit 120 may be, for example, an adduction and an abduction of the foot about the z-axis (yaw), a pronation and a supination of the foot about the y-axis (pitch), and a bending and a stretching of the foot about x-axis (roll).

Here, in order to sufficiently acquire features included in walking, it is desirable that time series data of angular velocity in three axial directions and acceleration in three directions include data of a period corresponding to at least one walking cycle. One walking cycle is described with reference to Fig. 6. Fig. 6 is a conceptual diagram illustrating a walking cycle. Fig. 6 schematically illustrates motion of the right foot and the left foot of the user 4 for one walking cycle. The normalized time in the figure indicates the time normalized so that the length of one walking cycle is 100. That is, the normalized time 0 in the figure is the moment at which the right foot lands, the normalized time 50 in the figure is the moment at which the left foot lands, and the normalized time 100 in the figure is the moment at which the right foot lands again. A period from the normalized time 0 to 100 is one walking cycle.

Further, a period in which the foot lands is referred to as a stance period, and a period in which the foot leaves the ground is referred to as a swing period. More specifically, for example, the stance period of the right foot is a period from the moment at which the heel of the right foot lands (at the time of landing) to the moment at which the toe of the right foot leaves the ground (at the time of departure), and generally occupies a period of about 60% of one walking cycle. The swing period of the right foot is a period from the moment when the toe of the right foot leaves the ground to the moment when the heel of the right foot lands, and generally occupies a period of about 40% of one walking cycle. As illustrated in Fig. 6, during walking, the stance period and the swing period are alternately repeated. Further, the phase of the stance period and the phase of the swing period are opposite between the right foot and the left foot.

In step S102, the coordinate system transforming unit 131 performs coordinate system transformation of angular velocity in three axial directions and acceleration in three directions. A coordinate system with respect to angular velocity and acceleration output by the IMU 12 is an inertial coordinate system. The coordinate system transforming unit 131 transforms the angular velocity and acceleration coordinate system into a coordinate system with respect to the foot of the user 4. Thus, the coordinate system of the angular velocity and the acceleration can be made suitable for calculating the rotation angle of the foot. The transformation of the coordinate system is realized, for example, by multiplying the base vector of the inertial coordinate system by the direction cosine matrix E using the Euler angle and rotating the base vector.

An example of transformation of the coordinate system by the direction cosine matrix E is described more specifically. In a case where the base vector of the inertial coordinate system is $[x_i, y_i, z_i]$, and the base vector of the coordinate system with respect to the foot is $[x_b, y_b, z_b]$, a conversion formula between them is expressed by the following equation (1).

[Math. 1]

$$\begin{bmatrix} x_b \\ y_b \\ z_b \end{bmatrix} = \begin{bmatrix} E_{11} & E_{12} & E_{13} \\ E_{21} & E_{22} & E_{23} \\ E_{31} & E_{32} & E_{33} \end{bmatrix} \begin{bmatrix} x_i \\ y_i \\ z_i \end{bmatrix} = E \begin{bmatrix} x_i \\ y_i \\ z_i \end{bmatrix} \qquad (1)$$

In a case where angle acquired by rotating the base vector of the inertial coordinate system by angles of $\psi$ (psi), $\theta$ (theta), and $\varphi$ (phi) in the order of z, y, and x is an Euler angle of the coordinate system transformation, the direction cosine matrix E is expressed by the following equation (2).

[Math. 2]

$$E = \begin{bmatrix} 1 & 0 & 0 \\ 0 & \cos\phi & \sin\phi \\ 0 & -\sin\phi & \cos\phi \end{bmatrix} \begin{bmatrix} \cos\theta & 0 & -\sin\theta \\ 0 & 1 & 0 \\ \sin\theta & 0 & \cos\theta \end{bmatrix} \begin{bmatrix} \cos\psi & \sin\psi & 0 \\ -\sin\psi & \cos\psi & 0 \\ 0 & 0 & 1 \end{bmatrix}$$

$$= \begin{bmatrix} \cos\theta\cos\psi & \cos\theta\sin\psi & -\sin\theta \\ \sin\phi\sin\theta\cos\psi - \cos\phi\sin\psi & \sin\phi\sin\theta\sin\psi + \cos\phi\cos\psi & \sin\phi\cos\theta \\ \cos\phi\sin\theta\cos\psi + \sin\phi\sin\psi & \cos\phi\sin\theta\sin\psi - \sin\phi\cos\psi & \cos\phi\cos\theta \end{bmatrix} \qquad (2)$$

Note that the calculation method used for conversion of the coordinate system is merely an example, and other calculation methods may be used. For example, a calculation method using a quaternion may be applied.

In step S103, the angle calculation unit 132 calculates the angle between the foot of the user 4 and the ground from the angular velocity in the three axial directions and the acceleration in the three directions after being transformed into the coordinate system with respect to the foot of the user 4. As a specific example of this process, there is a method in which angular velocity in three axial directions and acceleration in three directions are input to a Madgwick filter (Non Patent Literature 1), and a rotation angle in three axial directions of the foot is output as an output. The rotation angles in the three axial directions acquired by the Madgwick filter are the angles of adduction or abduction of the foot, the angle of pronation or supination of the foot, and the angle of bending or stretching of the foot. In a case where information on the rotation angle is not necessary in the extraction of the feature amount described later, step S103 may be omitted.

In step S104, the walking cycle identification unit 133 identifies one walking cycle from time series data of angular velocity, acceleration, and rotation angle. Since substantially the same motion is repeated for each step during walking, one walking cycle can be identified by detecting periodicity of time series data. For example, one walking cycle can be identified based on the appearance time of the peak or dip of the time series data, the frequency of the peak included in the frequency spectrum acquired by Fourier-transforming the time series data, or the like.

In step S105, the feature amount calculation unit 134 extracts a feature amount used for identifying the user 4 from the time series data of at least one walking cycle. The extracted feature amount is stored in the storage unit 150. The extraction of the feature amount is described with reference to two specific examples.

First, a first example is described with reference to Fig. 7. Fig. 7 is a graph illustrating an example of time series data of acceleration in the width direction of the foot. The horizontal axis of Fig. 7 represents time, and the vertical axis of Fig. 7 represents acceleration in the width direction. The unit G illustrated in Fig. 7 is a unit of acceleration based on the standard gravitational acceleration (about 9.8 m/s$^2$).

Three graphs of the different line types illustrate the acceleration in the width direction measured from three different subjects (subject 1, subject 2, subject 3). As understood from Fig. 7, in any of the three subjects, a steep peak appears at the beginning of the swing period, and a steep dip appears at the end of the swing period. In addition, the differences between the three graphs are large in the swing period, and are significantly large particularly in the vicinity of the peak and dip described above. Therefore, the subject can be identified by extracting the feature amount from the vicinity of the peak in the beginning of the swing period and the vicinity of the dip in the end of the swing period in the time series data of acceleration in the width direction of the foot.

Next, a second example is described with reference to Fig. 8. Fig. 8 is a graph illustrating an example of time series data of an adduction/abduction angle of the foot. The horizontal axis of Fig. 8 represents time, and the vertical axis of Fig. 8 represents the adduction/abduction angle.

Three graphs of the different line types illustrate the adduction/abduction angles of the feet measured from three different subjects (subject 1, subject 2, subject 3). As understood from Fig. 8, in any of the three subjects, a steep dip appears at the beginning of the swing period, and a steep peak appears at the end of the swing period. The differences between the three graphs are significantly large in the vicinity of the peak and dip. Therefore, the subject can be identified by extracting the feature amount from the vicinity of the dip in the beginning of the swing period and the vicinity of the peak of the end of the swing period in the time series data of acceleration in the width direction of the foot.

The reason why the feature appears in the vicinity of the beginning and end of the swing period is described. At the beginning of the swing period (that is, when the foot leaves the ground) during human walking, the toe of the foot faces downward. At the end of the swing period (that is, when the foot lands on the ground), the toe of the foot faces upward. At these timings, the foot is likely to swing in the adduction/abduction direction, so that the fluctuation in the acceleration in the width direction and the adduction/abduction angle becomes large. Since an individual difference is likely to occur in this fluctuation, as illustrated in Fig. 7 and Fig. 8, the difference between subjects is large, and the fluctuation is suitable for extracting a feature amount. Since these peaks and dips occur at least once in one walking cycle, time series data of at least one walking cycle is acquired and feature amount extraction is performed in the present example embodiment.

Note that the feature amount may be extracted from time series data other than the above-described example. Specifically, the feature amount may be extracted from time series data such as acceleration in the longitudinal direction of the foot, acceleration in the vertical direction of the foot, pronation/supination angle of the foot, and bending/stretching angle of the foot. Further, the feature amount may be extracted from data acquired secondarily by performing calculation such as time integration on the time series data (secondary data). Examples of the secondary data include the velocity in three directions of the foot, the trajectories in three directions of the foot, the walking speed, the step width, the landing time, the leaving time of the foot, the length of the stance period, and the length of the swing period. When the IMU 12 is provided on both feet of the user 4, the feature amount may be extracted from the difference, ratio, or the like of the time series data acquired from both feet or the secondary data. Further, a peak or dip may be extracted from the above-described time series data or secondary data, and the appearance time, intensity, or the like of the peak or dip may be used as a feature amount. Further, statistical amount acquired by performing statistical calculations such as a sum, an average value, a difference, a ratio, and a product on the appearance time, intensity, and the like of a peak or dip may be extracted as feature amounts. The feature amount extracted in this process may include a plurality of elements, in other words, the feature amount extracted in the present process may be a feature amount vector.

In step S106, the identification unit 140 identifies the user 4 based on the extracted feature amount. This identification process may be a process of determining whether or not the user 4 is the same person as a person registered in the gait measurement device 1 in advance (registrant). A specific example of this process is to compare the acquired feature amount with the feature amount of the registrant to calculate a score indicating a certainty factor, and to determine whether or not the user 4 is the same person as the registrant based on whether or not the score exceeds a predetermined threshold value.

In the process of identifying the user 4 from the feature amount performed by the identification unit 140, a trained model generated in advance by machine learning and stored in the storage unit 150 is used. Examples of algorithms used for machine learning include decision trees, random forests, support vector machines, neural networks, deep learning, logistic regression, k-nearest neighbor algorithm (K-NN), ensemble learning for classification method, discriminant analysis, or the like. Further, generation of a trained model by machine learning (training process) is performed in the gait measurement device 1, the information communication terminal 2, or the server 3 using sample data prepared in advance.

The training process for generating a trained model used for personal identification in step S106 is described in more detail. This process is performed in advance in the gait measurement device 1, the information communication terminal 2, or the server 3 prior to the process of Fig. 5. In the description of the present example embodiment, it is assumed that the training process is performed in the server 3.

Fig. 9 is a flowchart illustrating an example of training process performed by the server 3 according to the present example embodiment. The process of Fig. 9 is performed prior to the personal identification process at the time of shipment from a factory, calibration before the user 4 uses the gait measurement device 1, or the like.

In step S201, the server 3 acquires sample data for training. This sample data may be, for example, one in which a personal identification label (user identifier (ID), name, nickname, or the like) for identifying a person is associated with a feature amount vector acquired by the processing from step S101 to step S105. The personal identification label is attached in advance by the user 4, the administrator of the personal identification system, or the like. More specifically, by causing the user 4 to actually walk a predetermined distance after being input his/her personal identification label to the gait measurement device 1 and causing the gait measurement device 1 to acquire data, sample data in which feature amount vectors and personal identification labels are associated with each other can be created.

In step S202, the server 3 performs machine learning on the sample data as labeled training data. As a result, a trained model is generated in which a person is appropriately identified with respect to the input of the feature amount vector and the identification information is output.

In step S203, the server 3 stores the trained model in the flash memory 204. Thereafter, the server 3 provides the trained model to the gait measurement device 1. Specifically, the server 3 transmits the trained model to the information communication terminal 2. The information communication terminal 2 causes the gait measurement device 1 to install the received trained model as software for processing in the identification unit 140.

Fig. 10 is a table schematically illustrating the correspondence relation between a feature vector and a personal identification label acquired by the training process. As illustrated in Fig. 10, a personal identification label is determined corresponding to a feature amount vector including "maximum value of the acceleration in the width direction in the swing period", "minimum value of the adduction/abduction angle in the swing period", "peak value of the acceleration in the vertical direction in the swing period", and the like. In other words, the trained model acquired by the training process has a function of outputting a personal identification label as a response variable when a feature amount vector is input as an explanatory variable.

Hereinafter, the results of actually performing personal identification using the personal identification system of the first example embodiment are described as Example 1 and Example 2.

[Example 1]

In the present example, motion information during walking was measured for nine subjects, and walking data was acquired. A large number of feature amount vectors were extracted from these walking data to create data groups for training and validation. In the present example, cross-validation was performed using these data groups. Specifically, 15% of the data groups randomly selected were used as validation data, and the remaining 85% of the data were used as training data. That is, a trained model was generated using 85% of the data of the data groups, and the recognition rate of the trained model was validated using the remaining 15% of the validation data.

Fig. 11 is a table illustrating results of cross-validation using these data groups. The "predicted class" in the table is a class of a subject determined by the personal identification system of the first example embodiment, and the "true class" is an actual class indicating a subject. The class numbers "1" to "9" in the table indicate subject numbers. For example, in the prediction performed by the personal identification system for 185 data groups whose true class is "3", the personal identification system correctly predicted the class "3" for 184 of the 185 data groups. In contrast, an incorrect class "5" was predicted for one of 185. As illustrated in Fig. 11, in the personal identification system of the first example embodiment, the subject could be correctly determined at a high correct answer rate of 99.5% or more.

[Example 2]

In the present example, motion information during walking was measured for ten subjects, and walking data was acquired. A large number of feature vectors were extracted from these walking data to generate a trained model. Then, the recognition rate of the trained model was validated for the walking data of another day of the same subject. The recognition rate changes depending on the value of the certainty factor set as a threshold value for determining whether or not the person is the identical person, and the value of the certainty factor was 0.9 in this validation. The algorithm for machine learning used in the present example embodiment was deep learning.

Fig. 12 is a graph illustrating a result of validation of a recognition rate. In the graph of Fig. 12, the vertical axis represents the recognition rate, and the horizontal axis represents the subject number. As illustrated in Fig. 12, in the personal identification system of the first example embodiment, the subject could be determined at a high recognition rate of about 85%.

As described above, according to the present example embodiment, the information processing device 11 capable of suitably extracting the feature amount used for personal identification is provided. Further, a personal identification device and a personal identification system capable of performing personal identification with high accuracy by using the feature amount extracted by the information processing device 11 are provided.

In recent years, biometric authentication using biometric information has been widely used in personal authentication. Examples of the biological information include a face, a fingerprint, a voiceprint, and an iris. However, depending on where biometric authentication is performed, biometric authentication using them may not be effective.

For example, in facilities requiring wearing of protective clothes, masks, or the like, such as food plants, it is difficult to use authentication techniques using them because parts for acquiring biological information such as a face and a fingerprint are not exposed to the outside. Further, in facilities in which safety and health are severely required, such as in chemical plants, installation of a device for biometric authentication may be limited. In a facility where security is severely required, such as a power plant, authentication may be desired without informing the subject that biometric authentication is being performed. In addition, in a facility such as a care facility, where although it is required to continue authentication of a subject at all times, a blind spot exists in a biometric authentication device such as a camera. In addition, when it is necessary to always identify a subject, it is difficult to use an authentication method which requires the subject to perform an action on an input device such as a camera, a sensor, or a microphone.

Thus, depending on the location where biometric authentication is performed and the constraints required for biometric authentication, biometric authentication using a face, a fingerprint, a voiceprint, iris, or the like may not be effective.

In contrast, the biometric information included in the gait can be acquired even if a part such as a face or a fingerprint of the subject is not exposed to the outside. Further, the authentication device using the gait can be installed in the shoe, so that it is not necessary to install the biometric authentication device outside, and it is also possible to perform the biometric authentication without informing the subject that the biometric authentication is being performed. The gait can be measured at all times, and it is not necessary to request actions other than walking in the measurement. Therefore, the personal identification system of the present example embodiment can perform personal identification with high accuracy even in a place where biometric authentication such as a face, a fingerprint, a voiceprint, or an iris is not effective.

[Second Example Embodiment]

A personal identification system of the present example embodiment differs from that of the first example embodiment in that it has a function of converting time series data from a time domain to a frequency domain. Hereinafter, differences from the first example embodiment are mainly described, and description of common parts is omitted or simplified.

Fig. 13 is a functional block diagram of the information processing device 11 according to the present example embodiment. The feature amount extracting unit 130 further includes a data conversion unit 135 in addition to the elements described in the first example embodiment. The data conversion unit 135 has a function of converting time series data from the time domain to the frequency domain. Specifically, the data conversion unit 135 converts time series data acquired in the time domain into data in the frequency domain (frequency spectrum) by performing data conversion such as Fourier transform. The algorithm used for the transformation may be, for example, a fast Fourier transform.

The CPU 111 realizes the function of the data conversion unit 135 by loading a program stored in the ROM 113, the flash memory 114, or the like into the RAM 112 and executing the program. The function of the data conversion unit 135 may be provided in the information communication terminal 2 or the server 3, or may be provided in the IMU 12.

Fig. 14 is a flowchart illustrating an example of personal identification processing performed by the gait measurement device 1 according to the present example embodiment. Description of steps common to those in the first example embodiment is omitted.

In step S107, the data conversion unit 135 converts the time series data acquired in the time domain into the frequency domain by performing Fourier transform or the like on at least one walking cycle.

In step S108, the feature amount calculation unit 134 extracts a feature amount used for identifying the user 4 from the frequency spectrum acquired by the data conversion unit 135. The extracted feature amount is stored in the storage unit 150. The extraction of the feature amount is described with reference to a specific example.

Fig. 15 is a graph illustrating an example of a frequency spectrum of acceleration in the width direction of the foot. The horizontal axis of Fig. 15 represents the frequency normalized in units of "time/walk cycle", and the vertical axis of Fig. 15 represents the vibration intensity acquired by Fourier-transforming the acceleration in the width direction of the foot.

Fig. 16 is a graph illustrating an example of a frequency spectrum of an adduction/abduction rotation angle of a foot. The horizontal axis of Fig. 16 represents the frequency normalized in units of "time/walk cycle", and the vertical axis of Fig. 16 represents the vibration intensity acquired by Fourier-transforming the adduction/abduction angle of the foot.

In Fig. 15 and Fig. 16, three graphs of different line types illustrate spectra of vibration intensities acquired from three different subjects (subject 1, subject 2, and subject 3) . As can be understood from Fig. 15 and Fig. 16, in the case of three subjects, although the peak frequencies are similar on the low frequency side (e.g., in the range of less than 7 times/walking cycle), there is a difference in the peak height. Further, on the high frequency side (e.g., a range of equal to or more than 7 times /walking cycle), a difference is observed in both the shape and the height of the peak, but the vibration intensity is small. Therefore, the subject can be identified by extracting the height of some peaks on the low frequency side, the integral value of the vibration intensity on the high frequency side, or the like as feature amounts.

Further, parameters acquired by performing four arithmetic operations on such as peak height and integration value may be extracted as feature amounts, or a plurality of feature amounts may be extracted as feature amount vectors.

Fig. 17 is a table schematically illustrating a correspondence relation between a feature amount vector and a personal identification label acquired by the training process using the feature amount vector. As illustrated in Fig. 17, personal identification labels are determined corresponding to feature amount vectors including "main vibration peak intensity in the lateral direction", "main vibration peak intensity in the adduction/abduction", "integral value of high frequency components in the lateral direction", and the like. In other words, the trained model acquired by the training process has a function of outputting a personal identification label as a response variable when a feature amount vector is input as an explanatory variable.

Hereinafter, the results of actually performing personal identification using the personal identification system of the second example embodiment are described as Example 3 and Example 4.

[Example 3]

In the present example, motion information during walking was measured for ten subjects, and walking data was acquired. A large number of feature amount vectors were extracted from these walking data to create data groups for training and validation. In the present example, cross-validation was performed using these data groups. Specifically, 15% of the data groups randomly selected were used as validation data, and the remaining 85% of the data were used as training data. That is, a trained model was generated using 85% of the data of the data group, and the recognition rate of the trained model was validated using the remaining 15% of the validation data.

Fig. 18 is a table illustrating results of cross-validation using these data groups. The "predicted class" in the table is a class of a subject determined by the personal identification system of the second example embodiment, and the "true class" is an actual class indicating a subject. The class numbers "1" to "10" in the table indicate subject numbers. For example, in the prediction performed by the personal identification system for 168 data groups whose true class is "3", the personal identification system correctly predicted the class "3" for 165 of the 168 data groups. In contrast, for three out of 168, incorrect classes "1", "7" and "8" were predicted. As illustrated in Fig. 18, in the personal identification system of the second example embodiment, the subject could be correctly determined at a high correct answer rate of 98.5% or more.

[Example 4]

In the present example, motion information during walking was measured for ten subjects, and walking data was acquired. A large number of feature vectors were extracted from these walking data to generate a trained model. Then, the recognition rate of the trained model was validated for the walking data of another day of the same subject. The recognition rate changes depending on the value of the certainty factor set as a threshold value for determining whether or not the person is the same person, and the value of the certainty factor was 0.6 in this validation. The algorithm for machine learning used in the present example was deep learning.

Fig. 19 is a graph illustrating a result of validation of a recognition rate. In the graph of Fig. 19, the vertical axis represents the recognition rate, and the horizontal axis represents the subject number. As illustrated in Fig. 19, in the personal identification system of the second example embodiment, the subject could be determined at a high recognition rate of about 85%.

As described above, also in the present example embodiment, as in the first example embodiment, the information processing device 11 capable of suitably extracting the feature amount used for personal identification is provided. Further, a personal identification device and a personal identification system capable of performing personal identification with high accuracy by using the feature amount extracted by the information processing device 11 are provided.

The device or system described in the above example embodiments can also be configured as in the following third example embodiment.

[Third Example Embodiment]

Fig. 20 is a functional block diagram of the information processing device 61 according to the third example embodiment. The information processing device 61 includes an acquisition unit 611 and a feature amount extracting unit 612. The acquisition unit 611 acquires motion information of a foot of a user measured by a motion measurement device provided on the foot. The feature amount extracting unit 612 extracts a feature amount used for identifying the user from time series data of at least one walking cycle included in the motion information.

According to the present example embodiment, the information processing device 61 capable of suitably extracting the feature amount used for personal identification is provided.

[Modified Example embodiments]

The present invention is not limited to the example embodiments described above, and may be suitably modified within the scope of the present invention. For example, an example in which a part of the configuration of one example embodiment is added to another example embodiment or an example in which a part of the configuration of one example embodiment is replaced with another example embodiment is also an example embodiment of the present invention.

In the above-described example embodiments, the motion measurement device including the angular velocity sensor that measures the angular velocity in the three axial directions and the acceleration sensor that measures the acceleration in the three directions is used, but sensors other than these may also be used. For example, a magnetic sensor that detects geomagnetism by detecting magnetism in three directions to identify an azimuth may be further used. Even in this case, the same processing as the above-described example embodiments can be applied, and the accuracy can be further improved.

Although the personal identification process is performed inside the gait measurement device 1 in the above-described example embodiments, this function may be provided in the information communication terminal 2. In this case, the information communication terminal 2 functions as a personal identification device.

Although the feature amount is extracted from the time series data in the first example embodiment and the feature amount is extracted from the frequency spectrum in the second example embodiment, feature amounts may be extracted from both the time series data and the frequency spectrum.

Although the acceleration and the angular velocity are measured in the above-described example embodiment, when sufficient accuracy is acquired by only one of the acceleration and the angular velocity, measurement of the other can be omitted. In this case, one of the angular velocity sensor and the acceleration sensor of the IMU 12 can be omitted, and the cost of the component can be reduced.

A processing method in which a program for operating the configuration of the above-described example embodiments is recorded in a storage medium so as to implement the functions of the above-described example embodiments, the program recorded in the storage medium is read as code, and the program is executed in a computer is also included in the scope of each example embodiment. That is, a computer-readable storage medium is also included in the scope of the example embodiments. Further, not only the storage medium in which the above program is recorded, but also the program itself is included in each example embodiment. In addition, one or more components included in the above-described example embodiments may be a circuit such as an application specific integrated circuit (ASIC), a field programmable gate array (FPGA) configured to implement the functions of each component.

As the storage medium, for example, a floppy (registered trademark) disk, a hard disk, an optical disk, a magneto-optical disk, a compact disk (CD)-ROM, a magnetic tape, a nonvolatile memory card, or a ROM can be used. Further, the scope of each example embodiment is not limited to the case where the processing is executed by the program alone recorded in the storage medium, and a case where the processing is executed by operating on an operating system (OS) n cooperation with the functions of other software and extension board is also included in the scope of each example embodiment.

The service realized by the functions of the above-described example embodiments may be provided to the user in the form of a software as a service (SaaS).

It should be noted that the above-described example embodiments are merely examples of embodying the present invention, and the technical scope of the present invention should not be limitedly interpreted by these. That is, the present invention can be implemented in various forms without departing from the technical idea or the main features thereof.

The whole or part of the example embodiments disclosed above can be described as, but not limited to, the following supplementary notes.

(Supplementary note 1)

An information processing device comprising:

an acquisition unit configured to acquire motion information of a foot of a user measured by a motion measurement device provided on the foot; and
a feature amount extracting unit configured to extract a feature amount used for identifying the user from time series data of at least one walking cycle included in the motion information.

(Supplementary note 2)

The information processing device according to supplementary note 1, wherein the motion information includes at least one of acceleration and angular velocity.

(Supplementary note 3)

The information processing device according to supplementary note 2, wherein the feature amount extracting unit extracts the feature amount from the time series data of at least one of the acceleration and the angular velocity.

(Supplementary note 4)

The information processing device according to supplementary note 2 or 3, wherein the feature amount extracting unit includes a coordinate system transforming unit configured to transform a coordinate system of at least one of the acceleration and the angular velocity included in the motion information into a coordinate system with respect to the foot.

(Supplementary note 5)

The information processing device according to any one of supplementary notes 2 to 4, wherein the feature amount extracting unit includes an angle calculation unit configured to calculate a rotation angle of the foot using the acceleration and the angular velocity.

(Supplementary note 6)

The information processing device according to supplementary note 5, wherein the angle calculation unit calculates the rotation angle using a Madgwick filter.

(Supplementary note 7)

The information processing device according to supplementary note 5 or 6, wherein the feature amount extracting unit extracts the feature amount from the time series data of the rotation angle.

(Supplementary note 8)

The information processing device according to any one of supplementary notes 1 to 7, wherein the motion measurement device is provided at a position corresponding to an arch of the foot.

(Supplementary note 9)

The information processing device according to any one of supplementary notes 1 to 8, wherein the feature amount extracting unit extracts the feature amount after converting the time series data from a time domain to a frequency domain.

(Supplementary note 10)

The information processing device according to any one of supplementary notes 1 to 9, wherein the feature amount extracting unit extracts a feature amount used for identifying the user from a feature within a swing period in the time series data.

(Supplementary note 11)

A personal identification device configured to identify the user based on a feature amount extracted by the information processing device according to any one of supplementary notes 1 to 10.

(Supplementary note 12)

A personal identification system comprising:

the information processing device according to any one of supplementary notes 1 to 10;
an identification unit configured to identify the user based on the feature amount; and
the motion measurement device.

(Supplementary note 13)

An information processing method comprising:

acquiring motion information of a foot of a user measured by a motion measurement device provided on the foot; and extracting a feature amount used for identifying the user from time series data of at least one walking cycle included in the motion information.

(Supplementary note 14)

A storage medium storing a program that causes a computer to perform:

acquiring motion information of a foot of a user measured by a motion measurement device provided on the foot; and extracting a feature amount used for identifying the user from time series data of at least one walking cycle included in the motion information.

[Reference Signs List]

1 gait measurement device
2 information communication terminal
3 server
4 user
5 shoe
11, 61 information processing device
12 IMU
13 battery
111, 201 CPU
112, 202 RAM
113, 203 ROM
114, 204 flash memory
115, 205 communication I/F
116 IMU control device
120, 611 acquisition unit
130, 612 feature amount extracting unit
131 coordinate system transforming unit
132 angle calculation unit
133 walking cycle identification unit
134 feature amount calculation unit
135 data conversion unit
140 identification unit
150 storage unit
160 communication unit
206 input device
207 output device

**Claims**

1. An information processing device comprising:

    an acquisition unit configured to acquire motion information of a foot of a user measured by a motion measurement device provided on the foot; and
    a feature amount extracting unit configured to extract a feature amount used for identifying the user from time series data of at least one walking cycle included in the motion information.

2. The information processing device according to claim 1, wherein the motion information includes at least one of acceleration and angular velocity.

3. The information processing device according to claim 2, wherein the feature amount extracting unit extracts the feature amount from the time series data of at least one of the acceleration and the angular velocity.

4. The information processing device according to claim 2 or 3, wherein the feature amount extracting unit includes a coordinate system transforming unit configured to transform a coordinate system of at least one of the acceleration and the angular velocity included in the motion information into a coordinate system with respect to the foot.

5. The information processing device according to any one of claims 2 to 4, wherein the feature amount extracting unit includes an angle calculation unit configured to calculate a rotation angle of the foot using the acceleration and the angular velocity.

6. The information processing device according to claim 5, wherein the angle calculation unit calculates the rotation angle using a Madgwick filter.

7. The information processing device according to claim 5 or 6, wherein the feature amount extracting unit extracts the feature amount from the time series data of the rotation angle.

8. The information processing device according to any one of claims 1 to 7, wherein the motion measurement device is provided at a position corresponding to an arch of the foot.

9. The information processing device according to any one of claims 1 to 8, wherein the feature amount extracting unit extracts the feature amount after converting the time series data from a time domain to a frequency domain.

10. The information processing device according to any one of claims 1 to 9, wherein the feature amount extracting unit extracts a feature amount used for identifying the user from a feature within a swing period in the time series data.

11. A personal identification device configured to identify the user based on a feature amount extracted by the information processing device according to any one of claims 1 to 10.

12. A personal identification system comprising:

the information processing device according to any one of claims 1 to 10;
an identification unit configured to identify the user based on the feature amount; and
the motion measurement device.

13. An information processing method comprising:

acquiring motion information of a foot of a user measured by a motion measurement device provided on the foot; and
extracting a feature amount used for identifying the user from time series data of at least one walking cycle included in the motion information.

14. A storage medium storing a program that causes a computer to perform:

acquiring motion information of a foot of a user measured by a motion measurement device provided on the foot; and
extracting a feature amount used for identifying the user from time series data of at least one walking cycle included in the motion information.

# FIG. 1

# FIG. 2

| | | |
|---|---|---|
| 111 | CPU | |
| 112 | RAM | |
| 113 | ROM | |
| 114 | FLASH MEMORY | |
| 115 | COMMUNICATION I/F | |
| 116 | IMU CONTROL DEVICE | |
| 12 | IMU | |
| 13 | BATTERY | |

# FIG. 3

2

| | |
|---|---|
| 201 | CPU |
| 202 | RAM |
| 203 | ROM |
| 204 | FLASH MEMORY |
| 205 | COMMUNICATION I/F |
| 206 | INPUT DEVICE |
| 207 | OUTPUT DEVICE |

# FIG. 4

11

12

120 — ACQUISITION UNIT

IMU

130

FEATURE AMOUNT EXTRACTING UNIT

131 — COORDINATE SYSTEM TRANSFORMING UNIT

132 — ANGLE CALCULATION UNIT

133 — WALKING CYCLE IDENTIFICATION UNIT

134 — FEATURE AMOUNT CALCULATION UNIT

140 — IDENTIFICATION UNIT

150 — STORAGE UNIT

160 — COMMUNICATION UNIT

2

INFORMATION COMMUNICATION TERMINAL

# FIG. 5

START

ACQUIRE ACCELERATION
AND ANGULAR VELOCITY — S101

TRANSFORM A COORDINATE
OF ACCELERATION AND
ANGULAR VELOCITY — S102

CALCULATE AN ANGLE
BETWEEN THE SOLE OF THE
FOOT AND THE GROUND — S103

IDENTIFY ONE WALKING CYCLE
FROM TIME SERIES DATA — S104

EXTRACT A FEATURE AMOUNT
FROM TIME SERIES DATA IN
ONE WALKING CYCLE — S105

IDENTIFY THE USER BASED
ON THE FEATURE AMOUNT — S106

END

# FIG. 6

# FIG. 7

# FIG. 8

# FIG. 9

```
        ┌──────────────┐
        │    START     │
        └──────────────┘
               │
               ▼
   ┌───────────────────────────┐
   │   ACQUIRE SAMPLE DATA     │──── S201
   └───────────────────────────┘
               │
               ▼
   ┌───────────────────────────┐
   │  PERFORM A MACHINE        │
   │  LEARNING                 │
   │  USING SAMPLE DATA AS     │──── S202
   │  LABELED TRAINING DATA    │
   └───────────────────────────┘
               │
               ▼
   ┌───────────────────────────┐
   │  STORE THE TRAINED MODEL  │──── S203
   └───────────────────────────┘
               │
               ▼
        ┌──────────────┐
        │     END      │
        └──────────────┘
```

## FIG. 10

| EXPLANATORY VARIABLE | | | | RESPONSE VARIABLE |
|---|---|---|---|---|
| MAXIMUM VALUE OF THE ACCELERATION IN THE WIDTH DIRECTION IN THE SWING PERIOD | MINIMUM VALUE OF THE ADDUCTION/ABDUCTION ANGLE IN THE SWING PERIOD | PEAK VALUE OF THE ACCELERATION IN THE VERTICAL DIRECTION IN THE SWING PERIOD | · · | PERSONAL IDENTIFICATION LABEL |
| X1 | Y1 | Z1 | · · | ID1 |
| X2 | Y2 | Z2 | · · | ID2 |
| ⋮ | ⋮ | ⋮ | ⋮ | ⋮ |

EP 3 977 926 A1

# FIG. 11

FIG. 12

# FIG. 13

11

120 — ACQUISITION UNIT

12
IMU

130

FEATURE AMOUNT EXTRACTING UNIT

131 — COORDINATE SYSTEM TRANSFORMING UNIT

132 — ANGLE CALCULATION UNIT

133 — WALKING CYCLE IDENTIFICATION UNIT

134 — FEATURE AMOUNT CALCULATION UNIT

135 — DATA CONVERSION UNIT

140 — IDENTIFICATION UNIT

150 — STORAGE UNIT

160 — COMMUNICATION UNIT

2
INFORMATION COMMUNICATION TERMINAL

# FIG. 14

```
            ┌─────────────┐
            │    START    │
            └─────────────┘
                   │
                   ▼
    ┌──────────────────────────────┐
    │   ACQUIRE ACCELERATION        │───S101
    │   AND ANGULAR VELOCITY        │
    └──────────────────────────────┘
                   │
                   ▼
    ┌──────────────────────────────┐
    │   TRANSFORM A COORDINATE OF   │───S102
    │   ACCELERATION AND ANGULAR    │
    │   VELOCITY                    │
    └──────────────────────────────┘
                   │
                   ▼
    ┌──────────────────────────────┐
    │   CALCULATE AN ANGLE BETWEEN  │───S103
    │   THE SOLE OF THE FOOT AND THE│
    │   GROUND                      │
    └──────────────────────────────┘
                   │
                   ▼
    ┌──────────────────────────────┐
    │   IDENTIFY ONE WALKING CYCLE  │───S104
    │   FROM TIME SERIES DATA       │
    └──────────────────────────────┘
                   │
                   ▼
    ┌──────────────────────────────┐
    │   FOURIER-TRANSFORMING TIME   │───S107
    │   SERIES DATA ON AT LEAST ONE │
    │   WALKING CYCLE               │
    └──────────────────────────────┘
                   │
                   ▼
    ┌──────────────────────────────┐
    │   EXTRACT A FEATURE AMOUNT    │───S108
    │   FROM A SPECTRUM ACQUIRED BY │
    │   FOURIER-TRANSFORMING        │
    └──────────────────────────────┘
                   │
                   ▼
    ┌──────────────────────────────┐
    │   IDENTIFY THE USER BASED     │───S106
    │   ON THE FEATURE AMOUNT       │
    └──────────────────────────────┘
                   │
                   ▼
            ┌─────────────┐
            │     END     │
            └─────────────┘
```

# FIG. 15

# FIG. 16

## FIG. 17

| EXPLANATORY VARIABLE | | | | RESPONSE VARIABLE |
|---|---|---|---|---|
| MAIN VIBRATION PEAK INTENSITY IN THE LATERAL DIRECTION | MAIN VIBRATION PEAK INTENSITY IN THE ADDUCTION/ABDUCTION | INTEGRAL VALUE OF HIGH FREQUENCY COMPONENTS IN THE LATERAL DIRECTION | · · | PERSONAL IDENTIFICATION LABEL |
| X1 | Y1 | Z1 | · · | ID1 |
| X2 | Y2 | Z2 | · · | ID2 |
| ⋮ | ⋮ | ⋮ | ⋮ | ⋮ |

EP 3 977 926 A1

# FIG. 18

# FIG. 19

# FIG. 20

61

611 ACQUISITION UNIT

612 FEATURE AMOUNT
EXTRACTING UNIT

## INTERNATIONAL SEARCH REPORT

| International application No. |
|---|
| PCT/JP2019/021422 |

A.  CLASSIFICATION OF SUBJECT MATTER
Int. Cl. A61B5/117(2016.01)i

According to International Patent Classification (IPC) or to both national classification and IPC

B.  FIELDS SEARCHED

Minimum documentation searched (classification system followed by classification symbols)
Int. Cl. A61B5/117

Documentation searched other than minimum documentation to the extent that such documents are included in the fields searched
Published examined utility model applications of Japan      1922-1996
Published unexamined utility model applications of Japan    1971-2019
Registered utility model specifications of Japan            1996-2019
Published registered utility model applications of Japan    1994-2019

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y | US 2018/0089408 A1 (TAI KIN CHEUNG Jeffrey) 29 March 2018, paragraphs [0001]-[0072] (Family: none) | 1-14 |
| Y | US 5724265 A (HUTCHINGS, Lawrence, J.) 03 March 1998, column 3, line 56 to column 12, line 26 & WO 1997/021983 A1 | 1-14 |
| Y | WO 2017/158633 A1 (GIPSTECH S. R. L.) 21 September 2017, column 1, line 4 to column 27, line 21 (Family: none) | 5-12 |
| Y | US 2015/0112603 A1 (ZHONG, Yu) 23 April 2015, paragraphs [0002]-[0071] (Family: none) | 9-12 |

☒   Further documents are listed in the continuation of Box C.          ☐   See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14.08.2019 | 27.08.2019 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2019/021422 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2016-538980 A (ECOLE POLYTECHNIQUE FEDERALE DE LAUSANNE (EPFL)) 15 December 2016, paragraphs [0001]-[0217] & US 2016/0279418 A1, paragraphs [0001]-[0278] & WO 2015/063127 A1 & EP 2868343 A1 & CN 105792886 A | 10-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015153143 A

- JP 2016073366 A

**Non-patent literature cited in the description**

- **SEBASTIAN O. H. MADGWICK ; ANDREW J. L. HARRISON ; RAVI VAIDYANATHAN.** Estimation of IMU and MARG orientation using a gradient descent algorithm. *2011 IEEE International Conference on Rehabilitation Robotics,* 2011, 179-185